# EUROPEAN PATENT SPECIFICATION

(11) **EP 0 476 930 B1**
(45) Date of publication and mention of the grant of the patent: **12.11.1997**
(21) Application number: 91308338.2
(22) Date of filing: 12.09.1991
(51) Int. Cl.: C12Q 1/44, G01N 33/53, G01N 33/543, C12Q 1/68, G01N 33/546, C09B 7/02

(54) **Enzyme assays**
Enzymtests
Analyses enzymatiques

(30) Priority: 12.09.1990 JP 240018/90
(43) Date of publication of application: 25.03.1992
(73) Proprietor: Sankyo Company Limited, Tokyo (JP)
(72) Inventor: Tsuji, Akio, Tokyo (JP); Maeda, Masako, Tokyo (JP); Arakawa, Hidetoshi, Yokohama-shi, Kanagawa-gen (JP)
(74) Representative: Gibson, Christian John Robert

(56) References cited:
- EP-A- 0 243 126
- WO-A-89/09937
- GB-A- 1 128 371
- US-A- 4 942 127
- PROCEEDINGS OF THE ROYAL SOCIETY (Series B), vol. 148, 08 April 1958, London (GB); COTSON et al., pp. 506-519/

## Description

The present invention relates to assays, especially immunoassays, wherein a 3-O-indoxyl ester is converted to the corresponding indigo via the agency of a suitable enzyme.

Many types of assay technique are now known in the biological field, with new advances frequently being made. A recently developed field of particular interest is that of the enzyme immunoassay (EIA).

Enzyme immunoassay has the potential to be an extremely powerful technique. Essentially, all that is necessary for success is that an antibody can be raised which is specific for the substance to be assayed. An enzyme can then be cross-linked to the antibody, and the resulting labelled antibody exposed to a sample for assay. Once bound antibody has been separated from unbound, or free, antibody, the bound antibody can be assayed by the activity of its enzyme on a suitable substrate introduced after separation of the two phases.

One way to achieve bound/free (B/F) separation is to provide an antibody for the substance to be assayed which is bound to a solid phase (for example, a liquid chromatography column), introduce the sample, wash off unbound sample, and then introduce labelled antibody. As the substance for assay is then bound by two antibodies, this is known as a "sandwich" assay.

In an alternative technique, a second antibody, again bound to a suitable marker, such as an enzyme, is specific for the first antibody which binds the substance to be assayed. Thus, this technique is less direct, as it is assaying antibody bound to the substance to be assayed, rather than the substance itself.

One problem with this type of technique is the requirement to provide a suitable enzyme which can be assayed in such a fashion as to be definitive, that is, that there is no doubt that the assay results are indicative, whether qualitative or quantitative, of the substrate to be determined.

As will be appreciated, enzymes occur naturally and, where a bioassay is being carried out, it is entirely possible that there may be small, but significant, quantities of the enzyme, or its substrate, in the sample being assayed, which may distort the result.

One of the most sensitive assay techniques currently available in the field of enzyme immunoassay involves the use of enzymes which catalyse the production of hydrogen peroxide in the presence of the substrate. For example, the enzyme glucose oxidase may be linked to a suitable antibody for an assay, and the substrate, in this case glucose, introduced once the antibody has been separated from the sample, and the presence of hydrogen peroxide subsequently being determined to assay the quantity of target substance. Nevertheless, this assay technique still suffers from the drawbacks described above.

A more advantageous type of assay might suitably comprise a system wherein the enzyme is capable of acting on a synthetic substrate not normally encountered in a biological sample. An example of this type of assay includes the situation where bacteria, for example, are being tested for complementation. A suitable target for a complementation assay uses α-complementation in the lac operon. Introduction of a gene marked with the coding information for the first 146 amino acids of the β-galactosidase gene (lac Z) can convert a Lac⁻ mutant into a Lac⁺ mutant. A simple assay technique is then to incorporate X-gal (5-bromo-4-chloro-3-indolyl-β-D-galactopyranoside) into the substrate upon which the colonies are grown. Any Lac⁺-complemented colonies can then readily be counted, as they develop a blue color, through the action of reconstituted β-galactosidase on X-gal.

The results of such assays will generally be either positive or negative, but results lying somewhere inbetween may be detected by the presence of a faint coloration. In any event, there is no need to assay the result colorimetrically.

A similar type of assay is also known in the field of enzyme histochemistry to determine the presence of alkaline phosphatase in tissue samples. The substrate BCIP (5-bromo-4-chloro-3-indolyl phosphate), when acted upon by the enzyme alkaline phosphatase, develops a blue colour over a substantial period of time, as BCIP is first hydrolysed and then dimerised to form 5,5'-dibromo-4,4'-dichloroindigo. Suitable methods for the determination of enzymes located inside tissue cells by this technique are described in Enzyme Histochemistry: Asakura Pub. Inc. (1982).

The problem with this form of assay is that it is very slow and not particularly sensitive.

The use of BCIP alone has now been superseded by a similar assay, but which is conducted in the presence of either nitro blue tetrazolium (NBT) or tetranitro blue tetrazolium (TNBT). The assay is considerably faster, and gives rise to a blue colour through the production of a formazan, which can be assayed colorimetrically. This assay has proven to be considerably more sensitive, and its use has been extended to situations where greater degrees of accuracy are required.

Various techniques for the use of the BCIP/NBT assay have been described. For example, in Molecular Cloning [A Laboratory Manual, second edition, Sambrook, Frisch and Maniatis, (1989), Cold Spring Harbor Laboratory Press], there is described an immunoassay wherein alkaline phosphatase (ALP) is linked to an antibody in order to assay protein expression in transformed cells, and which is determined by the use of BCIP/NBT.

In an alternative technique, Dupont provide a kit comprising ALP covalently linked to a thymidine residue in a stretch of cDNA which can be used in standard hybridisation assays. The presence of ALP is then established as usual, by using BCIP/NBT to produce a colour.

However, a major problem still arises when using the BCIP/NBT technique, as it is not possible to assay very low concentrations of substances, such as might be found in individual cells. This is primarily because colorimetry must be used to assay the formazan produced, thus requiring a gross formation of dye, rather than measuring activity of enzyme at the molecular level.

Other assay techniques also exist, such as radioimmunoassay (RIA). In common with colorimetry, RIA tends to be used to assay the presence or absence of a given substance, at best offering a qualitative, rather than a quantitative, result. In addition, the use of the necessary radioisotopes is fraught with problems, prime amongst which are handling and disposal.

In Proceedings of the Royal Society [Series B, 148, 506-519 (1958)], there is described a mechanism for the production of indigo from 3-O-indoxyl derivatives. Essentially, an indoxyl ester is hydrolysed through the action of a suitable catalyst, such as a metal salt or enzyme. The resulting indoxyl then undergoes a reaction with atmospheric oxygen to form the corresponding indigo compound. It was established that hydrogen peroxide was formed as a result of this reaction, but that the quantity obtained was inconsistent. The report concluded that the production of hydrogen peroxide was an inconvenience (page 519, line 1 et seq). The detection of hydrolytic enzymes by the production of indigo from 3-O-indoxyl derivatives is also described in GB-A-1128371.

It has now been discovered that accurate enzyme assays, suitable for use in immunoassay techniques, are possible for enzymes associated with the dimerisation of indoxyl esters to form the corresponding indigo, with the assays utilising the production of hydrogen peroxide from this reaction, as hydrogen peroxide production is sufficiently consistent to be used to accurately calculate enzyme activity down to levels of 1 in 1000 compared to colorimetric techniques for the same enzymes.

Thus, in a first aspect of the present invention, there is provided a method for assaying a substance, preferably in a bioassay, wherein the assay comprises conversion of a 3-O-indoxyl ester, or esters, to a corresponding indigo via the agency of a suitable enzyme, or enzymes, preferably used as a label, characterised in that hydrogen peroxide production potential is determined.

It has been established that it is possible to accurately assay those enzymes associated with the dimerisation of indoxyl esters to form the corresponding indigo down to levels of accuracy not possible with prior art techniques. We have surprisingly found that the production of hydrogen peroxide from this reaction, rather than being a disadvantage as previously thought, is sufficiently consistent, often in stoichiometric amounts depending on the indoxyl, that it can be used to accurately calculate enzyme activity down to levels of 1 in 1000 compared to those measurable by colorimetric techniques.

Accordingly, it is possible to achieve the sensitivity of an enzyme immunoassay by employing an esterase useful to promote the dimerisation of indoxyl esters to form a corresponding indigo. The assay of the present invention, therefore, does not rely upon a natural substrate for the enzyme. Further, the sensitivity of a hydrogen peroxide assay is obtained without having to use standard substrates and which, in addition, can also be confirmed by colorimetric techniques.

The enzyme may be used in any suitable manner in the assays of the present invention, typically as a marker on an antibody, for example. Uses of enzymes capable of catalysing indigo production in assays are well known, and similar techniques can be employed in the present invention, except that hydrogen peroxide production potential is determined, rather than dye production.

Typically, the enzyme used in the assay of the present invention will be bound to a carrier which is, in some manner, interactive with the substance to be assayed, and non-interacting enzyme-carrier conjugate removed from the system. The amount of interaction will be representative of the amount or concentration of the assay substance. Thus, determination of enzyme activity can be used to calculate the amount of substance in the sample.

By "hydrogen peroxide production potential" is meant that amount of hydrogen peroxide which would be produced in a simple enzyme/substrate system. In most instances of the present invention, the actual production of hydrogen peroxide is measured, but it is possible to measure the potential, or ability, to produce hydrogen peroxide in some techniques.

Techniques wherein the hydrogen peroxide production potential is measured include the enzyme electrode techniques [for a review, see Robinson, G.A., et al., Clin. Chem., (1985), 31/9, 1449-52]. These techniques tend to involve a carbon electrode protected by a selective membrane, across which only selected solutes can pass, and have typically been used to detect glucose by the action of glucose oxidase, to give an indication of blood or urine sugar levels.

The action of glucose oxidase on glucose yields gluconolactone and hydrogen peroxide under normal circumstances but, in the enzyme electrode technique, this is by-passed by the presence of ferrocene or a similar compound. The ferrocene is reduced by the action of the enzyme, and no hydrogen peroxide is formed.

The reduced ferrocene can then cross the selectively permeable membrane and be reoxidised at the electrode surface. The resulting current can then be measured to give an indication of the amount of substance to be assayed which is present in the sample.

One suitable enzyme electrode technique employs a standard enzyme electrode, such as may be used for the detection of glucose, wherein an antibody specific for the substance to be assayed is bound to the surface of a selectively permeable membrane protecting the electrode, and:
A) If required, preparing a sample in a suitable liquid form, such as a suspension or solution;
B) Bringing the sample into contact with the bound antibody;
C) Preferably, washing the electrode to remove anything not retained by the bound antibody;
Either - D) (i) bringing a preparation of material specific for the substance to be assayed into contact with the electrode and, preferably, washing to remove material not retained by the bound antibody and (ii) bringing a preparation of the enzyme into contact with the electrode, the enzyme being bound to a carrier specific for the material;
   or
- E) Bringing a preparation of the enzyme into contact with the electrode, the enzyme being bound to a carrier specific for the substance to be assayed;
F) Washing the electrode to remove all substances not retained;
G) Bringing a preparation of a 3-O-indoxyl ester, together with ferrocene or similar substance, into contact with the electrode; and,
H) Measuring the current at the electrode.

The ferrocene may be incorporated into the membrane, rather than added separately, but it is preferred to add it separately, so that an excess of ferrocene can ensure that any current is not limited by restricted amounts of substrate.

Steps B-E may be performed in any order, provided that no washing takes place before step B is performed.

In an alternative embodiment of the enzyme electrode technique, the electrode may be mounted on a magnetisable substrate. Antibody specific for the assay substance may then be bound to magnetic particles, and mixed with a sample as well as a preparation of second antibody specific for the substance to be assayed. The mixture may then be brought into contact with the electrode and the magnetisable substrate magnetised; the electrode is then washed to remove any of the mixture not retained, the substrate remaining magnetised the while. Ferrocene, together with substrate for the enzyme, may then be added, and the current determined as before.

In any of the assays of the present invention, it is not necessary that the fixed substrate recognises the substance to assayed directly, nor that the enzyme carrier recognises the substance directly. Examples are given herein. The only requirement is for a direct chain of binding, such as by a number of suitably selected antibodies, to ensure that the enzyme activity measured is representative of the amount of the substance to be assayed in the sample. However, the fewer the number of links between support and enzyme, the better.

A direct assay of the present invention, in which the actual production of hydrogen peroxide is determined, may be characterised by a number of procedural steps as follows:
A) If required, preparing a sample in a suitable liquid form, such as a suspension or solution;
B) Bringing the sample into contact with a phase capable of selectively retaining the substance to be assayed;
C) Preferably, washing the phase to remove anything not retained by the phase;
Either - D) (i) bringing a preparation of material specific for the substance to be assayed into contact with the phase and, preferably, washing to remove material not retained by the phase and (ii) bringing a preparation of the enzyme into contact with the phase, the enzyme being bound to a carrier specific for the material
   or
- E) Bringing a preparation of the enzyme into contact with the phase, the enzyme being bound to a carrier specific for the substance to be assayed;
F) Washing the phase to remove all substances not retained;
G) Bringing a 3-O-indoxyl ester into contact with the phase and;
H) Assaying hydrogen peroxide, optionally after allowing reaction between the enzyme and the 3-O-indoxyl ester to proceed for a predetermined period.

In the above procedure, the enzyme is the enzyme of the present invention, and the substrate is a 3-O-indoxyl ester hydrolysable thereby. The "phase" is any suitable substrate or support, such a chromatography column or a filter which selectively retains the substance for assay under the washing conditions employed. The terms "selectively" and "specific" as used herein need not necessarily mean that only the substance to be assayed is retained or recognised, respectively, provided that at least one of the substances so defined, such as the phase, material or carrier, is so restricted.

For example, a nitrocellulose filter may be used to retain DNA, and it may only be necessary to assay for DNA in general, in which case the material of step D) may be a biotin-labelled λDNA probe, for example, and the enzyme may be linked to avidin. If a more specific assay is required, then the enzyme may be bound to a cDNA probe, for example. Similar considerations apply to other forms of assay, such as immunoassay.

In addition, various of the steps may be performed simultaneously or in a reverse order, provided that the desired effect is achieved. For example, steps B - E may be performed in any order, provided that no washing takes place before completion of any sequence of steps which is out of the specified sequence B, D/E. Steps G and H may also be performed simultaneously, although this is not preferred, as accuracy and sensitivity tend to be somewhat compromised.

An indirect, or competitive, assay of the present invention may be characterised by a number of procedural steps as follows:
A) If required, preparing a sample in a suitable liquid form, such as a suspension or solution;
B) Bringing the sample into contact with a phase capable of selectively retaining the substance to be assayed;
C) Bringing a predetermined amount of a stock preparation of labelled assay substance into contact with the phase in an amount sufficient to saturate the retention capabilities of the phase, and permitting time to allow labelled and unlabelled substance to compete;
D) Preferably washing the phase to remove anything not retained by the phase;
E) Where the label is not the enzyme, bringing a preparation of enzyme into contact with the phase, the enzyme being bound to a carrier specific for the label;
F) Washing the phase to remove all substances not retained;
G) Bringing a 3-O-indoxyl ester into contact with the phase and;
H) Assaying hydrogen peroxide, preferably after allowing the reaction to proceed for a predetermined period.

In the above procedure, the enzyme and substrate are as defined hereabove. In addition, the "label" is anything that serves to characterise the stock preparation of the substance which is to be assayed. Thus, it may be an antibody-substance complex, optionally covalently linked, or it may be a conjugate with the enzyme, for example. If it is an enzyme conjugate, then it is not necessary to perform step F.

Similar considerations apply to the competitive assay as to the direct assay. For example, steps B - E may be performed in any order, provided that no washing takes place before both steps B and C have been completed. Again, steps G and H may be performed simultaneously, although this is not preferred for the reasons given above.

Further, the enzyme-carrier conjugate need not be supplied so as to bind all of the substance or label, provided that the proportion of carrier without enzyme is known, to enable accurate determinations to be made. Such a consideration also applies to the direct assay. However, this is not generally preferred, as the amount of hydrogen peroxide will be diminished accordingly, requiring more sensitive determination techniques.

In the assays of the present invention, it is generally preferred to employ an excess of substances, such as antibodies, so as to ensure that the only limiting factor on the measurements made are as a result of the amount of the substance to be assayed.

Whilst the present invention is not bound by theory, it is believed that hydrogen peroxide is not generated by the effect of the direct action of the hydrolytic enzyme employed. It is thought that the enzyme serves to hydrolyse the 3-, and possibly the 1-, ester groupings, to yield the non-esterified indoxyl compound. Two molecules of indoxyl are then directly oxidised by two molecules of atmospheric oxygen to yield one molecule of indigo and two of hydrogen peroxide.

Thus, it will be appreciated that the enzymes which may be used in the assays of the present invention are only limited by the requirement that they must be able to hydrolyse a suitable 3-O-indoxyl ester. Further, as such esterase activity is commonly associated with the nature of the acyl group rather than the nature of the esterified molecule or substance, then any enzyme with hydrolytic activity is potentially useful in the assays of the present invention, always provided that a suitable 3-O-indoxyl ester can be made, or is available.

Typically, suitable enzymes include: alkaline phosphatase, β-D-galactosidase, esterase, acetylcholine esterase, alkyl sulphatase, sulphatase, β-D-glucosidase and β-D-glucuronidase.

Of the above enzymes, it is most preferred to use alkaline phosphatase or β-D-galactosidase, owing to their ready availability and stability.

The present invention also provides a kit for performing an assay as defined herein, comprising a 3-O-indoxyl ester, or esters, an enzyme or enzymes suitable for the conversion of said indoxyl ester or esters to the corresponding indigo and a means for determining the hydrogen peroxide production potential.

The 3-O-indoxyl ester useful in the assays of the present invention is preferably of the general formula (I): 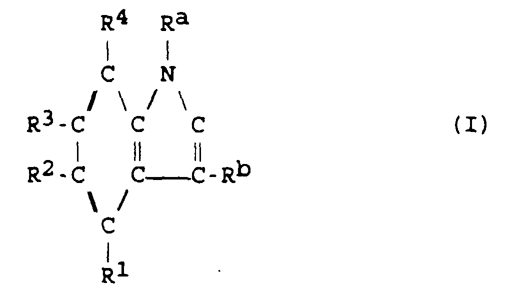 wherein
R¹, R², R³ and R⁴ may be the same or different and each represents a hydrogen atom or a small electrophilic moiety;
R^{a} represents a hydrogen atom or the acyl group of R^{b}; and
R^{b} represents any acyloxy group.

When any of R¹ - R⁴ represents a small electrophilic moiety, such a moiety is suitably selected from: cyano groups, amino groups, amino groups substituted with from 1 or 2 methyl or ethyl groups, trifluoromethyl groups, hydroxy groups, halogen atoms, methyl, ethyl, methoxy and ethoxy groups.

It is preferred that the substituents R¹ - R⁴ represent either hydrogen or halogen and, when halogen is represented, then bromine and/or chlorine are preferred.

It is possible for all of the substituents R¹ - R⁴ to represent electrophilic moieties as defined, but it is preferred that at least one represents hydrogen. It is also possible that all of the substituents R¹ - R⁴ represent hydrogen but, again, this is not preferred.

The most preferred compounds are those wherein two or three of the substituents R¹ - R⁴ represent hydrogen, and two or one represents a halogen atom. In particular, it is preferred that, where two of the substituents R¹ - R⁴ represent halogen, then one represents bromine, and the other represents chlorine. Where only one of the substituents R¹ - R⁴ represents halogen, then it is preferred that it represents bromine.

The most preferred positions on the compound of formula (I) for substitution are the 4- and 5- positions, corresponding to R¹ and R². Where only one of R¹ - R⁴ represents a group other than hydrogen, then it is preferred that this group is R² and, where two of the substituents R¹ - R⁴ represent a group rather than hydrogen, then it is preferred that these substituents are R¹ and R², those compounds wherein R² represents a 5-bromo- substituent being most preferred. It is possible to provide a bromo-substituent at the 6- position, but this tends not to provide stoichiometric production of hydrogen peroxide. Nevertheless, production of hydrogen peroxide is still sufficiently consistent to permit the assays of the present invention to be performed using it, with a suitable control or controls.

The substituent R^{a} may be either hydrogen or the acyl group corresponding to the acyloxy group specified for R^{b}. However, it is generally preferred that R^{a} represents a hydrogen atom.

When R^{a} represents an acyl group, then this acyl group should be hydrolysable by the same enzyme used to hydrolyse R^{b}. If this is not the case, then it may be necessary to employ further enzymes in the assays of the present invention, and it would not be possible to guarantee the quality of the results obtained. It may also prove difficult, or impossible, to suitably extrapolate from a reagent blank.

Preferred meanings for R^{a}, when R^{a} represents other than a hydrogen atom, are acyl groups derived from inorganic acids or simple organic acids.

When R^{a} represents an acyl group derived from an inorganic acid, then it is preferred that the acid is selected from: phosphoric acid, phosphonic acid, sulphuric acid, sulphonic acid and carbonic acid.

Where R^{a} represents an acyl group derived from a simple organic acid, then the preferred acids are carboxylic acids, such as: acetic acid, propanoic acid, butanoic acid and isomers thereof, pyruvic acid, sugar acids and L-amino acids.

R^{b} is not particularly limited, provided that it is a 3-O-acyl group. The only limitation on R^{b} is that it should be hydrolysable by an available enzyme. Accordingly, it will be appreciated that the 3-O-acyl groups will generally be those which occur in nature, or which correspond sufficiently closely to those occurring in nature to be hydrolysable by the relevant enzyme.

Typically available acyl groups include: phosphate, acetate, galactopyranosides, sulphate, glucuronate, glucopyranosides, fructopyranosides and mannopyranosides. It will also be appreciated that those acyl groups described for R^{a} above are suitable.

In general, the acyl groups represented by R^{b} will be derived from simple inorganic, or organic acids, or will be derived from such naturally occurring substances as amino acids and sugar acids. As described above, the residue represented by R^{b} need not necessarily be a naturally occurring residue, provided that it can be hydrolysed by an available enzyme.

It will also be appreciated that, when R^{b} and possibly R^{a}, represents a residue which may be salified, for example a phosphate group, then salts may be formed, and such salts are included within the ambit of the present invention. Again, such salts are not particularly limited, provided that they do not interfere with the assay of the present invention. Suitable salts will be those which balance the group R^{a} or R^{b}, so that, for example, the sulphate may form a salt with a base. Typical salts include the sodium and p-toluidine salts.

Particularly preferred indoxyl derivatives which may be used in accordance with the present invention include: 5-bromo-4-chloro-3-indolyl phosphate, 5-bromo-4-chloro-3-indolyl phosphate (disodium salt), 5-bromo-4-chloro-3-indolyl phosphate (p-toluidine salt), 3-indolyl phosphate (disodium salt), 3-indolyl phosphate (p-toluidine salt), 5-bromo-4-chloro-3-indolyl-β-D-galactopyranoside, 5-bromo-3-indolyl-β-D-galactopyranoside, 5-bromo-4-chloro-3-indolyl acetate, 5-bromo-4-chloro-3-indolyl-1,3-diacetate, 5-bromo-6-chloro-3-indolyl acetate, 5-bromo-4-chloro-3-indolyl sulphate (p-toluidine salt), 5-bromo-4-chloro-3-indolyl-β-D-glucopyranoside and 5-bromo-4-chloro-3-indolyl-β-D-glucuronate (MCHA [monocyclohexylammonium] salt).

As has been described hereinbefore, hydrogen peroxide is produced sufficiently consistently for highly accurate determinations of enzyme activity to be made.

Various methods are known for the determination of hydrogen peroxide, and have been well-described in the art. However, the methods suitable for use in the present invention are those which provide a measurement on a molecular basis, rather than on a volume or quantitative basis.

Suitable methods for determining hydrogen peroxide in accordance with the assay and step H of the direct procedures of the present invention, for example, include colorimetry, fluorimetry and chemiluminescence. Typical techniques are described in Molecular Cloning (supra) and Enzyme Immunoassay [3rd Ed., Igaku-Shoin, (1987)].

If colorimetry is employed to determine hydrogen peroxide, then appropriate reagents may include 1,2-diaminobenzene or 2,2'-amino-bis(3-ethylbenzothiazoline-6-sulphonic acid). These reagents undergo a reaction with hydrogen peroxide to produce intense colours easily assayable by a colorimeter. However, it will be appreciated that such a technique is unlikely to be able to reach the levels of sensitivity of fluorimetry or chemiluminescence.

Appropriate reagents for fluorimetry include p-hydroxyphenylpropionic acid, tyramine and homovaline, and the result of the reaction with hydrogen peroxide can be determined by a fluorimeter.

The most preferred technique is that of chemiluminescence, and appropriate reagents include: luminol, isoluminol, N-(4-aminobutyl)-N-ethyl luminol and other luminol derivatives, lucigenin, acridium, pyrogallol, oxalate, bis(2,4,6-trichlorophenyl)oxalate, bis(2,4-dinitrophenyl)oxalate, pentafluorophenyl oxalate and other oxalate derivatives, generally in association with a dye such as 8-anilinonaphthalene-1-sulphonic acid, fluorescein, rhodamine, pyrene or dansyl chloride. An oxidation catalyst, such as peroxidase, microperoxidase, potassium ferricyanide, potassium ferrocyanide or ferricyanide, is required before many of the above reagents can react with hydrogen peroxide to be determined by a luminescence reader, for example.

It will be appreciated that the activity of the enzyme must, in some manner, be representative of the amount of substance to be assayed in the sample. There is no restriction on how this may be effected, provided that the results of the assay can be used to determine the quantity of substance. The procedures described above give suitable examples of techniques which can be used to achieve the desired results.

In general terms, the enzyme may be so introduced to the system that it is either proportional or inversely proportional to the amount of substance to be assayed. If the enzyme activity is to be proportional to the substance to be assayed, then the assay will be conducted in such a manner that the amount of enzyme is determined by the amount of substance. This may take the form of fixing the substance to be assayed, binding enzyme to the fixed substance, washing off free enzyme, and then determining the activity of the enzyme, such as by the direct assay described above. When the amount of enzyme activity is inversely proportional, then the enzyme will be introduced into the system in such a manner as to be in competition with the substance to be assayed.

Obtaining enzyme activity representative of the amount of substance in the sample can be achieved in various manners. However, in general, the enzyme will be bound to some form of carrier which, itself, will recognise and bind the substance to be assayed, or which will compete for binding sites with the substance to be assayed.

It will commonly be the case that the enzyme carrier is an antibody. The resulting, labelled antibody can then be used in a known manner, such as in the direct assay procedure described. One example is ELISA (enzyme-linked immunosorbent assay), which may comprise passing an assay sample over antibody for the substance to be assayed, the antibody being linked to a solid support (solid-phase antibody - such as in step B of the direct and indirect assays of the present invention). At this point, it is preferred to wash the solid phase, in order to remove unbound sample (step C). However, washing need not occur until after the next step, which involves introducing the enzyme-labelled antibody (enzyme and carrier of step E), wherein the antibody is specific for the substance to be assayed. The solid phase is then washed to dispose of any unbound labelled antibody (step F), and a 3-O-indoxyl ester is then introduced (step G) and enzyme activity subsequently assayed (step H). This type of assay is known as a "sandwich" assay.

Other forms of assay involving labelled antibodies are also known, and these can involve, for example, the labelled antibody being specific for a type of antibody, such as mouse antibody. In such a situation, the mouse antibody may be specific for the substance to be assayed, and a first step of the assay involves binding mouse antibody to the substance (the material specific for the substance to be determined - step D) and separating bound and free antibody (B/F separation). Labelled antibody, such as goat anti-mouse antibody labelled with ALP, is then introduced (step E), washed to separate free antibody (step F), and the enzyme assayed as before.

Other suitable methods are described in Molecular Cloning (supra) and Enzyme Immunoassay (supra). These publications also provide suitable methods for crosslinking the enzyme to the antibody.

It will be appreciated that the antibodies used in the above assays are preferably monoclonal antibodies but, in some instances, it may be acceptable to use polyclonal antibodies. However, given the accuracy generally required, and the low concentration of substance to be assayed, then monoclonal antibodies will generally be chosen.

As indicated above, the carrier may be anything which will assist in regulating enzyme activity so that it is indicative of concentration of the assay substance. Thus, an alternative assay would involve preparing a labelled sample of the substance for assay, the label being an enzyme as described. The labelled substance can then be used in a competition assay, as described above, whereby the labelled substance and the substance in the sample to be assayed compete for binding on, for example, solid phase antibodies specific for the substance. The sample may be added before, after, or with the labelled substance, depending on how it is proposed to assay enzyme activity. However, it is generally preferred to add the sample at the same time as the labelled substance, in order to simplify calculations concerning the kinetics of competition.

In a preferred embodiment, an enzyme of the present invention is used to label an oligonucleotide probe. This probe can then be used directly in Southern blotting, and enzyme activity can be assayed directly on the nitrocellulose filter.

Alternatively, an oligonucleotide probe labelled with biotin may be employed to hybridise with the desired nucleotide sequence. After washing the filter to remove excess biotin-labelled probe, an enzyme-avidin conjugate may be introduced, washed to remove excess enzyme-avidin conjugate, and enzyme activity assayed as above. In this instance, it is not essential that the enzyme is directly bound to avidin, and it may be that avidin is used to label an antibody, enzyme being used to label a second antibody specific for the first. However, this is not preferred, as it introduces an unnecessary step into the technique.

There is no particular limitation on the nature of the substance to be assayed. Provided that some way can be established to moderate enzyme activity based on the concentration of the substance, then the criteria will have been met.

If the technique to be employed is associated with antibody, then the substance to be assayed should be antigenic. Thus, it is preferred that the substance to be assayed is a naturally occurring substance, such as one that may be found in the body. Suitable samples for assay may be taken from physiological fluids, or a suitably prepared biopsy. Suitable fluids include serum, plasma, urine and ascites.

Substances for assay are suitably substances which are capable of giving medical information if their presence and/or concentration can be determined. Examples of such substances include foetal protein, such as α-foetoprotein or CEA (carcinoembryonic antigen), hormones, such as peptide and steroid hormones, immunoglobulins, such as antiviral or antibacterial antibodies, and drugs.

The accompanying Examples further illustrate the present invention, but are not limiting thereupon in any way. Unless specified, all enzymes used in the Examples are of EIA grade.

### COMPARATIVE EXAMPLE

### Colorimetric Determination of Alkaline Phosphatase

To determine the minimum amount of alkaline phosphatase (ALP - EIA grade, Boehringer) which can be determined by the standard colorimetric technique (as described in Molecular Cloning, a Laboratory Journal, second edition, Eds. Sambrook, Frisch and Maniatis, 18.74), a number of dilutions of ALP was made up in a buffer comprising 0.1% BSA, 100mM MgCl₂ and 100mM Tris.HCℓ (pH 9.5).

Aliquots of 10 microlitres of ALP solution were dot plotted on a nitrocellulose membrane (BA85, Schlicher and Schuell), and immersed in BCIP/NBT (5-bromo-4-chloro-3-indolyl phosphate/nitro blue tetrazolium, Kirkegaard and Perry Laboratories Inc).

After developing for one hour, the dot plots were analysed for colour development, and it was established that the lowest amount of ALP detectable by this method is 100 pg/10 µl.

### EXAMPLE 1

### Detection of Alkaline Phosphatase by Chemiluminescence

Alkaline phosphatase was next assayed by chemiluminescence, to establish how low a concentration of the enzyme could be measured by this technique.

Dilutions of alkaline phosphatase (ALP) were prepared in a standard 0.1 M Tris.HCℓ buffer (containing 0.1% bovine serum albumin and 100 mM MgCℓ₂ [pH 9.5]). The dilutions prepared were from 100 pg/ml to 1 µg/ml. Ten µl of each solution were added to 100 µl lots of a 0.38 mM BCIP (Wako Pure Chem. Ind. Ltd.) solution in the standard Tris.HCℓ buffer.

The solutions were allowed to react for 1 hour at 37°C. The luminescence reagent (Tris-HCℓ buffer solution containing 0.12 mM of isoluminol and 0.5 µM of microperoxidase [pH 9.5]) was then added (500 µl), and the integrated luminescence intensity measured using a luminescence reader (Aloka).

A calibration curve for the assay was plotted, with ordinate and abscissa being the integrated intensity of luminescence (count) and concentration of alkaline phosphatase, respectively.

From the calibration curve, it is clear that levels of enzyme as low as 0.1 pg/10 µl are readily detectable.

Thus, it can be seen that the technique of the present invention is capable of providing useful results at concentrations of enzyme up to 1,000 times lower than can be used in conventional colorimetric techniques, such as are exemplified in the foregoing Comparative Example.

### EXAMPLE 2

### One-Step Determination of Alkaline Phosphatase

To investigate the possibility of assaying enzyme activity during hydrogen peroxide production, the substrate and chemiluminescence reagent were added simultaneously.

Dilutions of alkaline phosphatase (Boehringer) were prepared in the Tris.HCℓ buffer solution described in Example 1. The dilutions prepared were from 100 pg/ml to 1 µg/ml. Ten µl of each solution were added to 200 µl of the mixed solution of enzyme substrate and luminescence reagent (0.1 M Tris-HCℓ buffer solution containing 0.19 mM BCIP [p-toluidine salt - Wako Pure Chem. Ind. Ltd.], 0.25 mM of isoluminol, 5 U/ml horseradish peroxidase [Toyobo Co. Ltd.] and 100 mM of magnesium chloride [pH 9.5]), and the integrated luminescence intensity measured as in Example 1.

The change in integrated luminescence intensity for the various concentrations of alkaline phosphatase were plotted, ordinate and abscissa being the integrated luminescence intensity (count) and time respectively.

Essentially, it can be seen from the plots of integrated luminescence intensity and time that levels of enzyme as low as 10 pg/10 µl were detectable. The sensitivity is apparently inferior to that of the two-step assay Example 1, but demonstrates that one-step assays are feasible.

### EXAMPLE 3

### Detection of β-D-Galactosidase (β-Gal) by Chemiluminescence

In a manner similar to Example 1, β-D-galactosidase was assayed by chemiluminescence.

Dilutions of β-D-galactosidase (Boehringer) were prepared in phosphate buffer (0.01 M phosphate buffer solution containing 0.1% bovine serum albumin and 0.5 mM of magnesium chloride [pH 8.0]). The dilutions prepared were from 0.25 x 10⁻¹⁴ M to 1 x 10⁻¹⁰ M. Fifty µl of each solution were added to 100 µl lots of the above buffer solution containing 5-bromo-4-chloro-3-indolyl-β-D-galactopyranoside (X-Gal - buffer comprising 0.2 mM BCI Gal [Wako Pure Chem. Ind. Ltd.]), and maintained at 37°C.

After 2 hours, the luminescence reagent (Na₂CO₃ buffer solution containing 0.12 mM isoluminol and 0.5 µM of microperoxidase [pH 9.5]) was added and integrated luminescence intensity measured, as described in Example 1.

A calibration curve for the assay was plotted, ordinate and abscissa being the integrated luminescence intensity (count) and concentration of β-D-galactosidase respectively.

It can be seen from the calibration curve that levels of β-galactosidase as low as 2 x 10⁻¹⁹ mol/50 µl are detectable by this assay.

### EXAMPLE 4

### Detection of β-D-Glucosidase by Chemiluminescence

In a similar manner to Example 3, β-D-glucosidase was assayed by the chemiluminescence technique.

Dilutions of β-D-glucosidase (β-Glu, Boehringer) were prepared in acetate buffer (0.1 M acetate buffer solution containing 0.1% bovine serum albumin and 0.5 mM of magnesium chloride [pH 5.4]). The dilutions prepared were from 0.02 mU/ml to 200 mU/ml. Fifty µl of each solution were added to 100 µl lots of acetate buffer solution containing 5-bromo-4-chloro-3-indolyl-β-D-glucopyranoside (BCI Glu - buffer comprising 0.2 mM BCI Glu [Aldrich]), and maintained at 37°C.

After 2 hours, the reaction solution was mixed with the luminescence reagent (sodium carbonate buffer solution containing 0.12 mM of isoluminol and 0.5 µM of microperoxidase [pH 9.5]), and the integrated luminescence intensity measured as described in Example 1.

A calibration curve for the assay of β-D-glucosidase, was plotted, ordinate and abscissa being the integrated luminescence intensity (count) and concentration of β-D-glucosidase, respectively.

It can be seen from the calibration curve that levels of β-Glu as low as 5 x 10 ⁻¹⁶/50 µl (0.02 mU/ml) are detectable.

### EXAMPLE 5

### Calibration Curve of α-Foetoprotein (AFP) Using ALP-labelled Antibody

To establish that a protein, such as α-foetoprotein, can be assayed by a method of the present invention, a sandwich assay was performed.

Dilutions of α-foetoprotein (AFP) were prepared using 0.1 M phosphate buffer solution, so that 1000 ng/ml of AFP in phosphate buffer was diluted to obtain dilutions of 0.5, 1, 10, 100 and 1000 ng/ml.

Anti-AFP antibody coated tubes (AFP tubes) were prepared according to the procedure described in Fluorescent Enzyme Immunoassay Method of Thyroxine [Bunseki-Kagaku 34, 544-548 (1985)].

Thirty µl of each dilution were added to 270 µl of 0.01 M phosphate buffer solution, and introduced to an AFP tube.

After incubation for 15 minutes at 37°C, the AFP tube was washed three times with 1.5 ml of 0.01 M phosphate buffer solution to separate free AFP.

ALP-labelled anti-AFP antibodies were prepared according to the procedure described in Enzyme Immunoassay (supra). Three hundred µl of ALP-labelled anti-AFP antibody were added to the AFP tubes and, after incubation for 15 minutes at 37°C, the AFP tubes were washed three times, each time with 1.5 ml of 0.01 M phosphate buffer solution.

After the tubes had been washed, 300µl of buffer solution (0.1 M Tris-HCℓ buffer solution containing 0.1% bovine serum albumin and 100 mM of magnesium chloride [pH 9.5]) containing 0.38 mM of BCIP (Wako) were introduced into the tube. After 15 minutes at 37°C, 100 µl of the reaction solution were withdrawn and 100 µl luminescence reagent (CHES [2-cyclohexylaminoethane sulphonic acid] buffer solution [Dojin Kagaku] containing 0.2 mM of luminol and 60 µM of microperoxidase [pH 9.5]) mixed therewith, the integrated luminescence intensity then being measured using a chemiluminescence detector (Sankyo Co., Ltd., Tokyo). The luminescence intensity is given by the integration of output current value from a photomultiplier for 10 seconds. Units are represented in µA x sec.

A calibration curve for the assay was plotted, ordinate and abscissa being the integrated luminescence intensity (µA x sec) and concentration of α-foetoprotein respectively.

The resulting calibration curve demonstrates that levels as low as 1 ng/ml AFP can be measured with good sensitivity by this technique.

### EXAMPLE 6

### Calibration Curve for Non-Isotopic DNA Using Chemiluminescence

Dilutions of heat-denatured λ-DNA were prepared in deionised water. Dilutions were between 10 pg and 10 ng. Two hundred µl of each dilution was added to a microtitre plate and allowed to stand overnight at 4°C. After this time, the supernatant on the plates was removed.

Prehybridisation solution was then made up and added to each of the wells in amounts of 250µl. The prehybridisation solution consisted of 4 x ssc (ssc, as referred to herein, consists of 0.15 M NaCℓ, 0.015 M Na citrate - pH 7, multiples thereof defining multiples of concentration of NaCℓ and Na citrate with respect to the base solution), 0.064% PVP [polyvinyl-pyrrolidone], 0.064% Ficoll (Trade Mark), 0.064% bovine serum albumin and 50 µg/ml of heat-denatured bovine thymus DNA. The plates were then allowed to stand for 1 hour at 65°C.

After this time, the prehybridisation solution was removed, and 250 µl of hybridisation solution was added. The hybridisation solution comprised 4 x ssc, 0.064% PVP, 0.064% Ficoll (Trade Mark), 0.064% bovine serum albumin, 10% dextran sulphate, 50 µg/ml of heat-denanatured bovine thymus DNA and 1 µg/ml of biotin-labelled λ-DNA probe (prepared as described in Japanese Kokai, Hei No. 1-228500).

After reaction overnight at 65°C, the reaction solution was removed, and 250 µl of 2 x ssc added. This was allowed to react for 30 minutes at 65°C. Each well was then washed three times with 250 µl of phosphate buffer solution (pH 7.0) containing 0.9% NaCℓ.

After the wells had been washed, 150 µl of a diluted solution (20,000x) of the ALP-avidin conjugate (Bio-Yeda) was added to each well of the microtitre plate, and allowed to stand for 2 hours at room temperature.

After this time, each well was washed three times with 250 µl of phosphate buffer solution containing 0.9% NaCℓ. Subsequently, 150 µl of buffer solution (0.1 M Tris-HCℓ buffer solution containing 0.1% bovine serum albumin and 100 mM of magnesium chloride [pH 9.5]) containing 0.38 mM of BCIP (p-toluidine salt - Wako) was added to the reaction mixture and allowed to react for 1 hour at 37°C.

After this time, 500 µl of the luminescence reagent (Tris-HCℓ buffer solution containing 0.12 mM of isoluminol and 0.5 µM of microperoxidase [pH 9.5]) was added to 100 µl of the reaction solution and, from 15 seconds to 21 seconds after addition, the integrated luminescence intensity was measured for 6 seconds, using a luminescence reader (Aloka).

A calibration curve for the hybridization assay was plotted, ordinate and abscissa being the integrated luminescence intensity (count) and concentration of λ-DNA respectively.

The calibration curve shows that amounts of λ-DNA as low as 10 pg/well can be detected with high sensitivity.

### EXAMPLE 7

### Detection of a Specific Gene Sequence Using an ALP-Labelled Probe

Sample DNA (HSV-II plasmid, obtained from the SNAP [TM] kit, Molecular Biosystems, USA) is diluted with deionised water, boiled for 5 minutes and then rapidly cooled to form single strand DNA, which is then placed into wells in a polystyrene plate, each well containing 100 µl of DNA coating buffer (8 mM Na₂HPO₄, 1.5 mM KH₂PO₄, 137 mM NaCℓ, 2.7 mM KCℓ, 0.1 M MgCℓ₂.H₂O, pH unadjusted), and stirred. The sample is then subjected to physical adsorption on the polystyrene plate at 4°C overnight.

### Pre-hybridization

After removing the DNA solution from the well, 200µl of a hybridisation solution (5 x ssc, 0.5% BSA, 0.5% polyvinylpyrrolidone, pH unadjusted) are added and the reaction conducted on a water bath at 60°C for 20 minutes.

### Hybridisation

The hybridisation solution is removed from the well and 200µl of a hybridisation solution containing 50 µg/ml bovine thymus DNA and 1% (v/v) of SNAP ([Trade Mark] kit available from Molecular Biosystems, USA) Probe (alkaline-phosphatase-labelled HSV-II gene) added, the reaction being conducted on a water bath at 60°C for 20 minutes.

The hybridisation solution is then removed and washed twice, each time with 250 µl ssc, at room temperature for one minute and then twice again as before, this time at 60°C for 5 minutes.

After standing for 2 hours at room temperature, each well is washed three times with 250 µl of phosphate buffer solution containing 0.9% NaCl. Subsequently, 150 µl of buffer solution (0.1 M Tris-HCℓ buffer solution containing 0.1% bovine serum albumin and 100 mM of magnesium chloride [pH 9.5]) containing 0.38 mM of BCIP (p-toluidine salt - Wako) is added to the reaction mixture and allowed to react for 1 hour at 37°C.

After this time, 500 µl of the luminescence reagent (Tris-HCℓ buffer solution containing 0.12 mM of isoluminol and 0.5 µM of microperoxidase [pH 9.5]) is added to 100 µl of the reaction solution and, from 15 seconds to 21 seconds after addition, the integrated luminescence intensity is measured for 6 seconds, using a luminescence reader (Aloka).

## Claims

1. A method for assaying a substance, wherein the assay comprises conversion of a 3-O-indoxyl ester, or esters, to a corresponding indigo via the agency of a suitable enzyme, or enzymes, characterised in that hydrogen peroxide production potential is determined.

2. A method according to claim 1, comprising the steps:
A) If required, preparing a sample in a suitable liquid form, such as a suspension or solution;
B) Bringing the sample into contact with a phase capable of selectively retaining the substance to be assayed;
C) Preferably, washing the phase to remove anything not retained by the phase;
Either - D) (i) bringing a preparation of material specific for the substance to be assayed into contact with the phase and, preferably, washing to remove material not retained by the phase and (ii) bringing a preparation of the enzyme into contact with the phase, the enzyme being bound to a carrier specific for the material
or
- E) Bringing a preparation of the enzyme into contact with the phase, the enzyme being bound to a carrier specific for the substance to be assayed;
F) Washing the phase to remove all substances not retained;
G) Bringing a 3-O-indoxyl ester into contact with the phase and;
H) Assaying hydrogen peroxide, optionally after allowing reaction between the enzyme and the 3-O-indoxyl ester to proceed for a predetermined period.

3. A method according to claim 2, wherein the phase is washed to remove anything not retained thereby, after each of steps B, D(i)/E.

4. A method according to claim 2 or 3, wherein the phase comprises solid-phase antibodies or a filter for nucleotide sequence blotting.

5. A method according to any of claims 2 to 4, wherein at least one, and preferably all, of the phase, the material and the carrier is restricted specifically to the substance to be assayed.

6. A method according to any of claims 2 to 5, wherein steps B - E are performed in any order, provided that no washing takes place before step B has been performed.

7. A method according to any of claims 2 to 6, wherein steps G and H are performed simultaneously.

8. A method according to claim 1, comprising the steps:
A) If required, preparing a sample in a suitable liquid form, such as a suspension or solution;
B) Bringing the sample into contact with a phase capable of selectively retaining the substance to be assayed;
C) Bringing a predetermined amount of a stock preparation of labelled assay substance into contact with the phase in an amount sufficient to saturate the retention capabilities of the phase, and permitting time to allow labelled and unlabelled substance to compete;
D) Preferably washing the phase to remove anything not retained by the phase;
E) Where the label is not the enzyme, bringing a preparation of enzyme into contact with the phase, the enzyme being bound to a carrier specific for the label;
F) Washing the phase to remove all substances not retained;
G) Bringing a 3-O-indoxyl ester into contact with the phase and;
H) Assaying hydrogen peroxide, preferably after allowing the reaction to proceed for a predetermined period.

9. A method according to claim 8, wherein step D is performed.

10. A method according to claim 8 or 9, wherein the label is anything that serves to characterise the stock preparation of the substance to be assayed.

11. A method according to claim 10, wherein the label is a complex between a preparation of the substance to be assayed and an antibody, or a conjugate between the substance to be assayed and the enzyme.

12. A method according to any of claims 8 to 11, wherein the phase comprises solid-phase antibodies or a filter for nucleotide sequence blotting.

13. A method according to any of claims 8 to 12, wherein at least one, and preferably all, of the phase, the material and the carrier is restricted specifically to the substance to be assayed.

14. A method according to any of claims 8 to 13, wherein steps B - E are performed in any order, provided that no washing takes place before both steps B and C have been completed.

15. A method according to any of claims 8 to 14, wherein steps F and G are performed simultaneously.

16. A method according to claim 1, wherein an enzyme electrode is employed, the electrode having antibody specific for the substance to be assayed bound to an exposed surface of a selectively permeable membrane protecting the electrode, and:
A) If required, preparing a sample in a suitable liquid form, such as a suspension or solution;
B) Bringing the sample into contact with the bound antibody;
C) Preferably, washing the electrode to remove anything not retained by the bound antibody;
Either - D) (i) bringing a preparation of material specific for the substance to be assayed into contact with the electrode and, preferably, washing to remove material not retained by the bound antibody and (ii) bringing a preparation of the enzyme into contact with the electrode, the enzyme being bound to a carrier specific for the material;
or
- E) Bringing a preparation of the enzyme into contact with the electrode, the enzyme being bound to a carrier specific for the substance to be assayed;
F) Washing the electrode to remove all substances not retained;
G) Bringing a preparation of a 3-O-indoxyl ester, together with ferrocene or similar substance, into contact with the electrode; and,
H) Measuring the current at the electrode.

17. A method according to claim 16, wherein the electrode is washed after step B and, where appropriate, after step D(i).

18. A method according to claim 16, wherein steps B - E are performed in any order, provided that no washing takes place before step B is performed.

19. A method according to claim 1, wherein an enzyme electrode is used, the electrode being mounted on a magnetisable substrate, and:
A) Antibody specific for the assay substance is bound to magnetic particles;
B) The prepared particles of step A are mixed with a sample and with a preparation of second antibody specific for the substance to be assayed;
C) The mixture of step B is then brought into contact with the electrode and the magnetisable substrate magnetised;
D) Said electrode is washed to remove any of the mixture not retained, the substrate remaining magnetised the while;
E) Bringing a preparation of a 3-O-indoxyl ester, together with ferrocene or similar substance, into contact with the electrode; and,
F) The current at the electrode is determined.

20. A method according to any preceding claim, wherein the 3-O-indoxyl ester has the general formula (I): 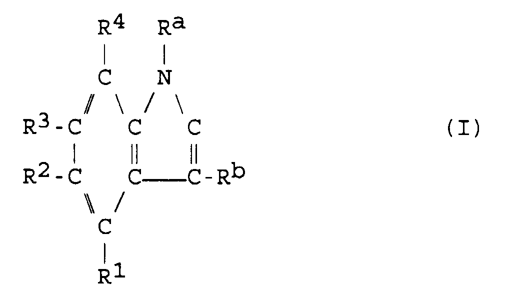 wherein
R¹, R², R³ and R⁴ may be the same or different and each represents a hydrogen atom or a small electrophilic moiety;
R^{a} represents a hydrogen atom or acyl group of R^{b}; and
R^{b} represents any acyloxy group.

21. A method according to claim 20, wherein at least one of R¹ - R⁴ represents a small electrophilic moiety.

22. A method according to claim 21, wherein the electrophilic moiety is a cyano group, an amino group, an amino group substituted with 1 or 2 methyl or ethyl groups, a trifluoromethyl group, a hydroxy group, a halogen atom, or a methyl, ethyl, methoxy or ethoxy group.

23. A method according to claim 20, wherein R¹ - R⁴ each represents hydrogen or halogen.

24. A method according to claim 21, wherein the electrophilic moiety is bromine or chlorine.

25. A method according to claim 20, wherein one or two of the substituents R¹ - R⁴ represents a halogen atom and the remainder represent hydrogen.

26. A method according to claim 20, wherein one of the substituents R¹ - R⁴ represents a bromine atom and the remainder represent hydrogen.

27. A method according to claim 20, wherein one of the substituents R¹ - R⁴ represents a bromine atom, one of the substituents R¹ - R⁴ represents a chlorine atom, and the remainder represent hydrogen.

28. A method according to any of claims 20 to 27, wherein R³ and R⁴ each represent hydrogen, one or both of R¹ and R² representing the electrophilic moiety and, when one of R¹ and R² does not represent halogen, then that substituent represents hydrogen.

29. A method according to any of claims 20 to 28, wherein R^{a} represents a hydrogen atom.

30. A method according to any of claims 20 to 29, wherein R^{a} represents an acyl group derived from an inorganic acid or a simple organic acid.

31. A method according to claim 30, wherein R^{a} is an acyl group derived from phosphoric acid, phosphonic acid, sulphuric acid, sulphonic acid or carbonic acid.

32. A method according to any preceding claim 30, wherein R^{a} is derived from acetic acid, propanoic acid, butanoic acid and isomers thereof, pyruvic acid, a sugar acid or an L-amino acid.

33. A method according to any of claims 20 to 32, wherein R^{b} is a phosphate, acetate, galactopyranoside, sulphate, glucuronate, glucopyranoside, fructopyranoside or a mannopyranoside.

34. A method according to any preceding claim, wherein the 3-O-indoxyl ester is a salt.

35. A method according to claim 34, wherein the salt is a p-toluidine salt, a mono- or di- sodium salt, or a monocyclohexyl ammonium salt.

36. A method according to any preceding claim, wherein the 3-O-indoxyl ester is the 5-bromo-4-chloro-3-indolyl phosphate, 5-bromo-4-chloro-3-indolyl phosphate disodium salt, 5-bromo-4-chloro-3-indolyl phosphate p-toluidine salt, 3-indolyl phosphate disodium salt, 3-indolyl phosphate p-toluidine salt, 5-bromo-4-chloro-3-indolyl-β-D-galactopyranoside, 5-bromo-3-indolyl-β-D-galactopyranoside, 5-bromo-4-chloro-3-indolyl acetate, 5-bromo-4-chloro-3-indolyl-1,3-diacetate, 5-bromo-6-chloro-3-indolyl acetate, 5-bromo-4-chloro-3-indolyl sulphate p-toluidine salt, 5-bromo-4-chloro-3-indolyl-β-D-glucopyranoside or the 5-bromo-4-chloro-3-indolyl-β-D-glucuronate monocyclohexyl ammonium salt.

37. A method according to any preceding claim, wherein the enzyme is alkaline phosphatase, β-D-galactosidase, esterase, acetylcholine esterase, alkyl sulphatase, sulphatase, β-D-glucosidase or β-D-glucuronidase.

38. A method according to any preceding claim, wherein the hydrogen peroxide is assayed by colorimetry, fluorimetry or preferably chemiluminescence.

39. A method according to any preceding claim 38, wherein the technique is colorimetry, and the reagent comprises 1,2-diaminobenzene or 2,2'-amino-bis-(3-ethylbenzothiazoline-6-sulphonic acid).

40. A method according to claim 38, wherein the technique is fluorimetry, and the reagent comprises p-hydroxyphenylpropionic acid, tyramine or homovaline.

41. A method according to any preceding claim 38, wherein the technique is chemiluminescence, and the reagent is luminol, isoluminol, N-(4-aminobutyl)-N-ethyl luminol or another luminol derivative, lucigenin, acridium, pyrogallol, oxalate, bis(2,4,6-trichloro-phenyl)oxalate, bis(2,4-dinitrophenyl)oxalate, pentafluorophenyl oxalate or another oxalate derivative, in association with a dye and an oxidation catalyst, where necessary.

42. A method according to any preceding claim 41, wherein the dye comprises 8-anilinonaphthalene-1-sulphonic acid, fluorescein, rhodamine, pyrene or dansyl chloride.

43. A method according to any preceding claim, wherein the enzyme is bound to a carrier therefor.

44. A method according to claim 43, wherein the carrier is an antibody, the substance to be assayed or an oligonucleotide probe.

45. A method according to any preceding claim, when performed on a physiological fluid or a suitably prepared biopsy.

46. A method according to any preceding claim, wherein the substance to be assayed is α-foetoprotein, carcinoembryonic antigen, a peptide hormone, a steroid hormone, an immunoglobulin or a drug.

47. A kit for performing an assay as defined in Claim 1 and comprising a 3-O-indoxyl ester, or esters, an enzyme or enzymes suitable for the conversion of said indoxyl ester or esters to the corresponding indigo and a means for determinining hydrogen peroxide production potential.

## Patentansprüche

1. Verfahren zum Nachweis einer Substanz, wobei der Nachweis die Umwandlung eines 3-O-Indoxylesters oder von Estern in eine entsprechende Indigo-Verbindung über die Einwirkung eines geeigneten Enzyms oder geeigneter Enzyme umfaßt, dadurch gekennzeichnet, daß das Wasserstoffperoxid-Bildungspotential bestimmt wird.

2. Verfahren nach Anspruch 1, das folgende Stufen umfaßt:
A) gegebenenfalls wird eine Probe in einer geeigneten flüssigen Form, wie einer Suspension oder Lösung, hergestellt;
B) die Probe wird in Kontakt mit einer Phase gebracht, die imstande ist, die Substanz, die nachgewiesen werden soll, selektiv zurückzuhalten;
C) vorzugsweise wird die Phase gewaschen, um alles zu entfernen, was nicht von der Phase zurückgehalten wird;
entweder: D) (i) eine Zubereitung von Material, das spezifisch für die Substanz ist, die nachgewiesen werden soll, wird mit der Phase in Kontakt gebracht und vorzugsweise wird gewaschen, um Material, das von der Phase nicht zurückgehalten wird, zu entfernen; und (ii) eine Zubereitung des Enzyms wird in Kontakt mit der Phase gebracht, wobei das Enzym an einen Träger gebunden ist, der spezifisch für das Material ist;
oder: E) eine Zubereitung des Enzyms wird in Kontakt mit der Phase gebracht, wobei das Enzym an einen Träger gebunden ist, der spezifisch für die Substanz ist, die nachgewiesen werden soll;
F) die Phase wird zur Entfernung aller Substanzen, die nicht zurückgehalten werden, gewaschen;
G) ein 3-O-Indoxylester wird in Kontakt mit der Phase gebracht; und
H) Wasserstoffperoxid wird nachgewiesen, wahlweise nach Ablauf einer Reaktion zwischen dem Enzym und dem 3-O-Indoxylester für eine festgelegte Zeitspanne.

3. Verfahren nach Anspruch 2, wobei die Phase nach jeder der Stufen B, D(i)/E gewaschen wird, um alles zu entfernen, was nicht davon zurückgehalten wird.

4. Verfahren nach Anspruch 2 oder 3, wobei die Phase Festphasenantikörper oder einen Filter für einen Nucleotidsequenz-Blot umfaßt.

5. Verfahren nach einem der Ansprüche 2 bis 4, wobei mindestens einer der Bestandteile Phase, Material und Träger und vorzugsweise alle diese Bestandteile spezifisch auf die Substanz beschränkt sind, die nachgewiesen werden soll.

6. Verfahren nach einem der Ansprüche 2 bis 5, wobei die Stufen B - E in einer beliebigen Reihenfolge durchgeführt werden, sofern keine Waschstufe erfolgt, bevor Stufe B durchgeführt worden ist.

7. Verfahren nach einem der Ansprüche 2 bis 6, wobei die Stufen G und H gleichzeitig durchgeführt werden.

8. Verfahren nach Anspruch 1, das folgende Stufen umfaßt:
A) gegebenenfalls wird eine Probe in einer geeigneten flüssigen Form, wie einer Suspension oder Lösung, hergestellt;
B) die Probe wird in Kontakt mit einer Phase gebracht, die imstande ist, selektiv die Substanz, die nachgewiesen werden soll, zurückzuhalten;
C) eine festgelegte Menge einer Stammzubereitung von markierter Nachweissubstanz wird in Kontakt mit der Phase in einer ausreichenden Menge gebracht, um das Rückhaltevermögen der Phase zu sättigen, und es wird eine ausreichende Zeit gelassen, um eine Konkurrenz von markierter und nicht markierter Substanz zu ermöglichen;
D) vorzugsweise wird die Phase gewaschen, um alles zu entfernen, was von der Phase nicht zurückgehalten wird;
E) wenn es sich bei der Markierung nicht um ein Enzym handelt, wird die Zubereitung des Enzyms in Kontakt mit der Phase gebracht, wobei das Enzym an einen Träger gebunden ist, der spezifisch für die Markierung ist;
F) die Phase wird gewaschen, um alle Substanzen, die nicht zurückgehalten werden, zu entfernen;
G) ein 3-O-Indoxylester wird in Kontakt mit der Phase gebracht; und
H) Wasserstoffperoxid wird nachgewiesen, vorzugsweise nach Ablauf der Reaktion für eine festgelegte Zeitspanne.

9. Verfahren nach Anspruch 8, wobei Stufe D durchgeführt wird.

10. Verfahren nach Anspruch 8 oder 9, wobei die Markierung ein beliebiges Material ist, das dazu dient, die Stammzubereitung der Substanz, die nachgewiesen werden soll, zu charakterisieren.

11. Verfahren nach Anspruch 10, wobei die Markierung ein Komplex zwischen einer Zubereitung der Substanz, die nachgewiesen werden soll, und einem Antikörper oder ein Konjugat zwischen der Substanz, die nachgewiesen werden soll, und dem Enzym ist.

12. Verfahren nach einem der Ansprüche 8 bis 11, wobei die Phase Festphasenantikörper oder einen Filter für einen Nucleotidsequenz-Blot umfaßt.

13. Verfahren nach einem der Ansprüche 8 bis 12, wobei mindestens einer der Bestandteile Phase, Material und Träger und vorzugsweise alle diese Bestandteile spezifisch auf die Substanz, die nachgewiesen werden soll, beschränkt sind.

14. Verfahren nach einem der Ansprüche 8 bis 13, wobei die Stufen B - E in einer beliebigen Reihenfolge durchgeführt werden, sofern keine Waschstufe durchgeführt wird, bevor die beiden Stufen B und C abgeschlossen sind.

15. Verfahren nach einem der Ansprüche 8 bis 14, wobei die Stufen F und G gleichzeitig durchgeführt werden.

16. Verfahren nach Anspruch 1, wobei eine Enzymelektrode eingesetzt wird, die einen Antikörper, der spezifisch für die Substanz, die nachgewiesen werden soll, gebunden an eine exponierte Oberfläche einer selektiv permeablen Membran, die die Elektrode schützt, aufweist, und das Verfahren folgende Stufen umfaßt:
A) gegebenenfalls wird eine Probe in einer geeigneten flüssigen Form, wie einer Suspension oder Lösung, hergestellt;
B) die Probe wird in Kontakt mit dem gebundenen Antikörper gebracht;
C) die Elektrode wird vorzugsweise gewaschen, um alles, was nicht von dem gebundenen Antikörper zurückgehalten wird, zu entfernen;
entweder: D) (i) eine Zubereitung von Material, das spezifisch für die Substanz ist, die nachgewiesen werden soll, wird mit der Elektrode in Kontakt gebracht, und vorzugsweise wird gewaschen, um Material, das von dem gebundenen Antikörper nicht zurückgehalten wird, zu entfernen; und (ii) eine Zubereitung des Enzyms wird in Kontakt mit der Elektrode gebracht, wobei das Enzym an einen Träger gebunden ist, der spezifisch für das Material ist;
oder: E) eine Zubereitung des Enzyms wird in Kontakt mit der Elektrode gebracht, wobei das Enzym an einen Träger gebunden ist, der spezifisch für die Substanz ist, die nachgewiesen werden soll;
F) die Elektrode wird gewaschen, um alle Substanzen, die nicht zurückgehalten werden, zu entfernen;
G) eine Zubereitung eines 3-O-Indoxylesters wird zusammen mit Ferrocen oder einer ähnlichen Substanz in Kontakt mit der Elektrode gebracht; und
H) der Strom an der Elektrode wird gemessen.

17. Verfahren nach Anspruch 16, wobei die Elektrode nach Stufe B und, wo dies in Betracht kommt, nach Stufe D(i) gewaschen wird.

18. Verfahren nach Anspruch 16, wobei die Stufen B - E in einer beliebigen Reihenfolge durchgeführt werden, sofern keine Waschstufe stattfindet, bevor Stufe B durchgeführt wird.

19. Verfahren nach Anspruch 1, wobei eine Enzymelektrode verwendet wird, die auf einem magnetisierbaren Substrat angeordnet ist, und das Verfahren folgende Stufen umfaßt:
A) Antikörper, der spezifisch für die Nachweissubstanz ist, wird an magnetische Teilchen gebunden;
B) die hergestellten Teilchen von Stufe A werden mit einer Probe und einer Zubereitung eines zweiten Antikörpers, der spezifisch für die Substanz ist, die nachgewiesen werden soll, gemischt;
C) das Gemisch von Stufe B wird dann in Kontakt mit der Elektrode und dem magnetisierten magnetisierbaren Substrat gebracht;
D) die Elektrode wird gewaschen, um alle Bestandteile des Gemisches, die nicht zurückgehalten werden, zu entfernen, wobei das Substrat währenddessen magnetisiert bleibt;
E) eine Zubereitung eines 3-O-Indoxylesters wird zusammen mit Ferrocen oder einer ähnlichen Substanz in Kontakt mit der Elektrode gebracht; und
F) der Strom an der Elektrode wird bestimmt.

20. Verfahren nach einem der vorstehenden Ansprüche, wobei der 3-O-Indoxylester die folgende allgemeine Formel (I) aufweist: 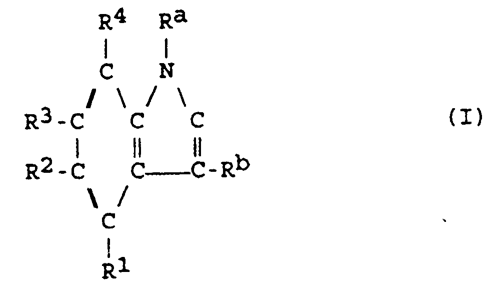 worin
R¹, R², R³ und R⁴ gleich oder verschieden sein können und jeweils ein Wasserstoffatom oder eine kleine elektrophile Gruppe darstellen;
R^{a} ein Wasserstoffatom oder eine Acylgruppe von R^{b} darstellt; und
R^{b} eine beliebige Acyloxygruppe darstellt.

21. Verfahren nach Anspruch 20, wobei mindestens einer der Reste R¹ - R⁴ eine kleine elektrophile Gruppe darstellt.

22. Verfahren nach Anspruch 21, wobei die elektrophile Gruppe eine Cyanogruppe, eine Aminogruppe, eine mit 1 oder 2 Methyl- oder Ethylgruppen substituierte Aminogruppe, eine Trifluormethylgruppe, eine Hydroxygruppe, ein Halogenatom oder eine Methyl-, Ethyl-, Methoxy- oder Ethoxygruppe ist.

23. Verfahren nach Anspruch 20, wobei jeder der Reste R¹ - R⁴ Wasserstoff oder Halogen darstellt.

24. Verfahren nach Anspruch 21, wobei die elektrophile Gruppe Brom oder Chlor ist.

25. Verfahren nach Anspruch 20, wobei einer oder zwei der Substituenten R¹ - R⁴ Halogenatome darstellen und die restlichen Wasserstoff darstellen.

26. Verfahren nach Anspruch 20, wobei einer der Substituenten R¹ - R⁴ ein Bromatom darstellt und die restlichen Wasserstoffatome darstellen.

27. Verfahren nach Anspruch 20, wobei einer der Substituenten R¹ - R⁴ ein Bromatom darstellt, einer der Substituenten R¹ - R⁴ ein Chloratom darstellt und die restlichen Wasserstoff darstellen.

28. Verfahren nach einem der Ansprüche 20 bis 27, wobei jeder der Reste R³ und R⁴ Wasserstoff darstellt, einer oder beide Reste R¹ und R² eine elektrophile Gruppe darstellen und, wenn einer der Reste R¹ und R² kein Halogen darstellt, dieser Substituent dann Wasserstoff darstellt.

29. Verfahren nach einem der Ansprüche 20 bis 28, wobei R^{a} ein Wasserstoffatom darstellt.

30. Verfahren nach einem der Ansprüche 20 bis 29, wobei R^{a} eine Acylgruppe darstellt, die von einer anorganischen Säure oder einer einfachen organischen Saure abgeleitet ist.

31. Verfahren nach Anspruch 30, wobei R^{a} eine Acylgruppe, die von Phosphorsäure, Phosphonsäure, Schwefelsäure, Sulfonsäure oder Kohlensäure abgeleitet ist, darstellt.

32. Verfahren nach Anspruch 30, wobei R^{a} von Essigsäure, Propansäure, Butansäure und Isomeren davon, Brenztraubensäure, einer Zuckersäure oder einer L-Aminosäure abgeleitet ist.

33. Verfahren nach einem der Ansprüche 20 bis 32, wobei R^{b} ein Phosphat, Acetat, Galactopyranosid, Sulfat, Glucuronat, Glucopyranosid, Fructopyranosid oder Mannopyranosid ist.

34. Verfahren nach einem der vorstehenden Ansprüche, wobei der 3-O-Indoxylester ein Salz ist.

35. Verfahren nach Anspruch 34, wobei das Salz ein p-Toluidinsalz, ein Mono- oder Dinatriumsalz oder ein Monocyclohexylammoniumsalz ist.

36. Verfahren nach einem der vorstehenden Ansprüche, wobei der 3-O-Indoxylester das 5-Brom-4-chlor-3-indolylphosphat, das 5-Brom-4-chlor-3-indolylphosphat-dinatriumsalz, das 5-Brom-4-chlor-3-indolylphosphat-p-toluidin-salz, das 3-Indolylphosphat-dinatriumsalz, das 3-Indolylphosphat-p-toluidinsalz, das 5-Brom-4-chlor-3-indolyl-β-D-galactopyranosid, das 5-Brom-3-indolyl-β-D-galactopyranosid, das 5-Brom-4-chlor-3-indolylacetat, das 5-Brom-4-chlor-3-indolyl-1,3-diacetat, das 5-Brom-6-chlor-3-indolylacetat, das 5-Brom-4-chlor-3-indolylsulfat-p-toluidin-salz, das 5-Brom-4-chlor-3-indolyl-β-D-glucopyranosid oder das 5-Brom-4-chlor-3-indolyl-β-D-glucuronat-monocyclohexylammoniumsalz ist.

37. Verfahren nach einem der vorstehenden Ansprüche, wobei das Enzym alkalische Phosphatase, β-D-Galactosidase, Esterase, Acetylcholinesterase, Alkylsulfatase, Sulfatase, β-D-Glucosidase oder β-D-Glucuronidase ist.

38. Verfahren nach einem der vorstehenden Ansprüche, wobei das Wasserstoffperoxid colorimetrisch, fluorimetrisch und vorzugsweise durch Chemolumineszenz nachgewiesen wird.

39. Verfahren nach Anspruch 38, wobei die Technik die Colorimetrie ist und das Reagenz 1,2-Diamonibenzol oder 2,2'-Amino-bis(3-ethylbenzothiazolin-6-sulfonsäure) umfaßt.

40. Verfahren nach Anspruch 38, wobei die Technik die Fluorimetrie ist und das Reagenz p-Hydroxyphenylpropionsäure, Tyramin oder Homovalin umfaßt.

41. Verfahren nach Anspruch 38, wobei die Technik die Chemolumineszenz ist und das Reagenz Luminol, Isoluminol, N-(4-Aminobutyl)-N-ethyl-luminol oder ein anderes Luminolderivat, Lucigenin, Acridium, Pyrogallol, Oxalat, Bis(2,4,6-trichlorphenyl)oxalat, Bis(2,4-dinitrophenyl)oxalat, Pentafluorphenyloxalat oder ein anderes Oxalatderivat in Verbindung mit einem Farbstoff und einem Oxidationskatalysator, sofern erforderlich, ist.

42. Verfahren nach Anspruch 41, wobei der Farbstoff 8-Anilinonaphthalin-1-sulfonsäure, Fluorescein, Rhodamin, Pyren oder Dansylchlorid umfaßt.

43. Verfahren nach einem der vorstehenden Ansprüche, wobei das Enzym an einen Träger dafür gebunden ist.

44. Verfahren nach Anspruch 43, wobei der Träger ein Antikörper, die Substanz, die nachgewiesen werden soll, oder eine Oligonucleotidsonde ist.

45. Verfahren nach einem der vorstehenden Ansprüche, wobei es mit einer physiologischen Flüssigkeit oder geeignet hergestellten Biopsieprobe durchgeführt wird.

46. Verfahren nach einem der vorstehenden Ansprüche, wobei die Substanz, die nachgewiesen werden soll, α-Foetoprotein, carcinoembryogenes Antigen, ein Peptidhormon, ein Steroidhormon, ein Immunglobulin oder ein Arzneistoff ist.

47. Reagenzsatz zur Durchführung des Nachweises gemäß der Definition in Anspruch 1, umfassend einen 3-O-Indoxylester oder Ester, ein Enzym oder Enzyme, die für die Umwandlung des Indoxylesters oder der Ester in die entsprechenden Indigoverbindungen geeignet sind, und Mittel zum Nachweis des Wasserstoffperoxid-Bildungspotentials.

## Revendications

1. Procédé pour analyser une substance, dans lequel l'essai comprend la conversion d'un ou plusieurs esters 3-O-indoxyliques en un indigo correspondant, à l'aide d'une ou plusieurs enzymes appropriées, caractérisé en ce que l'on détermine le potentiel de production de peroxyde d'hydrogène.

2. Procédé selon la revendication 1, comprenant les étapes suivantes :
A) si nécessaire, préparation d'un échantillon sous une forme liquide appropriée, telle qu'une suspension ou une solution ;
B) mise de l'échantillon en contact avec une phase pouvant retenir sélectivement la substance à analyser ;
C) de préférence lavage de la phase pour éliminer tout ce qui n'est pas retenu par la phase ;
soit
D) (i) mise d'une préparation d'une matière spécifique de la substance à analyser en contact avec la phase et, de préférence,lavage pour éliminer la matière non retenue par la phase et (ii) mise d'une préparation de l'enzyme en contact avec la phase, l'enzyme étant liée à un support spécifique de la matière
soit
E) mise d'une préparation de l'enzyme en contact avec la phase, l'enzyme étant liée à un support spécifique de la substance à analyser ;
F) lavage de la phase pour éliminer toutes les substances non retenues ;
G) mise d'un ester 3-O-indoxylique en contact avec la phase ; et
H) détermination du peroxyde d'hydrogène, éventuellement après avoir laissé la réaction entre l'enzyme et l'ester 3-O-indoxylique se poursuivre pendant une période prédéterminée.

3. Procédé selon la revendication 2, où la phase est lavée pour éliminer tout ce qu'elle ne retient pas après chacune des étapes B, D(i)/E.

4. Procédé selon la revendication 2 ou 3, où la phase comprend des anticorps en phase solide ou un filtre pour le transfert d'une séquence nucléotidique.

5. Procédé selon l'une quelconque des revendications 2 à 4, où au moins un, et de préférence tous, de la phase, de la matière et du support, sont limités spécifiquement à la substance à analyser.

6. Procédé selon l'une quelconque des revendications 2 à 5, où les étapes B à E sont effectuées dans un ordre quelconque, sous réserve qu'aucun lavage ne soit effectué avant la réalisation de l'étape B.

7. Procédé selon l'une quelconque des revendications 2 à 6, où les étapes G et H sont réalisées simultanément.

8. Procédé selon la revendication 1, comprenant les étapes suivantes :
A) si nécessaire, préparation d'un échantillon sous une forme liquide appropriée, telle qu'une suspension ou une solution ;
B) mise de l'échantillon en contact avec une phase susceptible de retenir sélectivement la substance à analyser ;
C) mise d'une quantité prédéterminée d'une préparation-mère de substance à analyser marquée en contact avec la phase, en une quantité suffisante pour saturer les capacités de rétention de la phase, en laissant un temps suffisant pour que la substance marquée et la substance non marquée entrent en compétition ;
D) de préférence lavage de la phase pour éliminer tout ce qui n'est pas retenu par la phase ;
E) lorsque le marqueur n'est pas l'enzyme, mise d'une préparation de l'enzyme en contact avec la phase, l'enzyme étant liée à un support spécifique du marqueur ;
F) lavage de la phase pour éliminer toutes les substances qui ne sont pas retenues ;
G) mise d'un ester 3-O-indoxylique en contact avec la phase ; et
H) détermination du peroxyde d'hydrogène, de préférence après avoir laissé la réaction se produire pendant une période prédéterminée.

9. Procédé selon la revendication 8, où l'étape D est effectuée.

10. Procédé selon la revendication 8 ou 9, où le marqueur est tout ce qui sert à caractériser la préparation-mère de la substance à analyser.

11. Procédé selon la revendication 10, où le marqueur est un complexe entre une préparation de la substance à analyser et un anticorps, ou un conjugué entre la substance à analyser et l'enzyme.

12. Procédé selon l'une quelconque des revendications 8 à 11, où la phase comprend des anticorps en phase solide ou un filtre pour le transfert d'une séquence nucléotidique.

13. Procédé selon l'une quelconque des revendications 8 à 12, où au moins un, et de préférence tous, de la phase, de la matière et du support, sont limités spécifiquement à la substance à analyser.

14. Procédé selon l'une quelconque des revendications 8 à 13, où les étapes B à E sont effectuées dans un ordre quelconque, sous réserve qu'aucun lavage ne soit effectué avant l'accomplissement des étapes B et C

15. Procédé selon l'une quelconque des revendications 8 à 14, où les étapes F et G sont réalisées simultanément.

16. Procédé selon la revendication 1, où on utilise une électrode enzymatique, l'électrode ayant un anticorps spécifique de la substance à analyser lié à une surface apparente d'une membrane à perméabilité sélective protégeant l'électrode, et qui comprend les étapes de :
A) si nécessaire, préparation d'un échantillon sous une forme liquide appropriée, telle qu'une suspension ou une solution ;
B) mise de l'échantillon en contact avec l'anticorps lié ;
C) de préférence, lavage de l'électrode pour éliminer tout ce qui n'est pas retenu par l'anticorps lié ;
soit
D) (i) mise d'une préparation d'une matière spécifique de la substance à analyser en contact avec l'électrode et de préférence lavage pour éliminer la matière non retenue par l'anticorps lié et (ii) mise d'une préparation de l'enzyme en contact avec l'électrode, l'enzyme étant liée à un support spécifique de la matière ;
soit
E) mise d'une préparation de l'enzyme en contact avec l'électrode, l'enzyme étant liée à un support spécifique de la substance à analyser ;
F) lavage de l'électrode pour éliminer toutes les substances non retenues ;
G) mise d'une préparation d'un ester 3-O-indoxylique avec du ferrocène ou une substance semblable en contact avec l'électrode ; et
H) mesure du courant au niveau de l'électrode.

17. Procédé selon la revendication 16, où l'électrode est lavée après l'étape B et s'il convient après l'étape D(i).

18. Procédé selon la revendication 16, où les étapes B à E sont effectuées dans un ordre quelconque, sous réserve qu'aucun lavage ne soit effectué avant l'accomplissement de l'étape B.

19. Procédé selon la revendication 1, où on utilise une électrode enzymatique, l'électrode étant montée sur un substrat aimantable, et :
A) un anticorps spécifique de la substance à analyser est lié à des particules magnétiques ;
B) les particules préparées dans l'étape A sont mélangées avec un échantillon et avec une préparation d'un second anticorps spécifique de la substance à analyser ;
C) le mélange de l'étape B est ensuite mis en contact avec l'électrode et le substrat aimantable aimanté ;
D) ladite électrode est lavée pour éliminer tout mélange non retenu, le substrat demeurant entre-temps aimanté ;
E) une préparation d'un ester 3-O-indoxylique avec du ferrocène ou une substance semblable est mise en contact avec l'électrode ; et
F) le courant au niveau de l'électrode est déterminé.

20. Procédé selon l'une quelconque des revendications précédentes, où l'ester 3-O-indoxylique répond à la formule générale (I) : 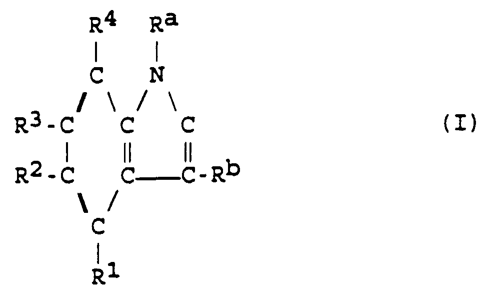 dans laquelle
R¹, R², R³ et R⁴ peuvent être identiques ou différents et représentent chacun un atome d'hydrogène ou un petit fragment électrophile ;
R^{a} représente un atome d'hydrogène ou un groupe acyle de R^{b} ; et
R^{b} représente un groupe acyloxy quelconque.

21. Procédé selon la revendication 20, où au moins un de R¹ à R⁴ représente un petit fragment électrophile.

22. Procédé selon la revendication 21, où le fragment électrophile est un groupe cyano, un groupe amino, un groupe amino substitué avec un ou deux groupes méthyle ou éthyle, un groupe trifluorométhyle, un groupe hydroxy, un atome d'halogène ou un groupe méthyle, éthyle, méthoxy ou éthoxy.

23. Procédé selon la revendication 20, où R¹ à R⁴ représentent chacun un atome d'hydrogène ou un atome d'halogène.

24. Procédé selon la revendication 21, où le fragment électrophile est un atome de brome ou de chlore.

25. Procédé selon la revendication 20, où un ou deux des substituants R¹ à R⁴ représentent un atome d'halogène et les autres représentent un atome d'hydrogène.

26. Procédé selon la revendication 20, où un des substituants R¹ à R⁴ représente un atome de brome et les autres représentent un atome d'hydrogène.

27. Procédé selon la revendication 20, où un des substituants R¹ à R⁴ représente un atome de brome, un des substituants R¹ à R⁴ représente un atome de chlore et les autres représentent un atome d'hydrogène.

28. Procédé selon l'une quelconque des revendications 20 à 27, où R³ et R⁴ représentent chacun un atome d'hydrogène, un de R¹ et R² ou les deux représentent le fragment électrophile et, lorsque un de R¹ et R² ne représentent pas un atome d'halogène, alors ce substituant représente un atome d'hydrogène.

29. Procédé selon l'une quelconque des revendications 20 à 28, où R^{a} représente un atome d'hydrogène.

30. Procédé selon l'une quelconque des revendications 20 à 29, où R^{a} représente un groupe acyle dérivé d'un acide minéral ou d'un acide organique simple.

31. Procédé selon la revendication 30, où R^{a} est un groupe acyle dérivé de l'acide phosphorique, de l'acide phosphonique, de l'acide sulfurique, de l'acide sulfonique ou de l'acide carbonique.

32. Procédé selon la revendication 30, où R^{a} dérive de l'acide acétique, de l'acide propanoïque, de l'acide butanoïque et de ses isomères, de l'acide pyruvique, d'un acide saccharique ou d'un L-aminoacide.

33. Procédé selon l'une quelconque des revendications 20 à 32, où R^{b} est un groupe phosphate, acétate, galactopyrannoside, sulfate, glucuronate, glucopyrannoside, fructopyrannoside ou mannopyrannoside.

34. Procédé selon l'une quelconque des revendications précédentes, où l'ester 3-O-indoxylique est un sel.

35. Procédé selon la revendication 34, où le sel est un sel de p-toluidine, un sel de mono- ou de disodium ou un sel de monocyclohexylammonium.

36. Procédé selon l'une quelconque des revendications précédentes, où l'ester 3-O-indoxylique est le phosphate de 5-bromo-4-chloro-3-indolyle, le sel disodique du phosphate de 5-bromo-4-chloro-3-indolyle, le sel de p-toluidine du phosphate de 5-bromo-4-chloro-3-indolyle, le sel disodique du phosphate de 3-indolyle, le sel de p-toluidine du phosphate de 3-indolyle, le 5-bromo-4-chloro-3-indolyl-β-D-galactopyrannoside, le 5-bromo-3-indolyl-β-D-galactopyrannoside, l'acétate de 5-bromo-4-chloro-3-indolyle, le 1,3-diacétate de 5-bromo-4-chloro-3-indolyle, l'acétate de 5-bromo-6-chloro-3-indolyle, le sel de p-toluidine du sulfate de 5-bromo-4-chloro-3-indolyle, le 5-bromo-4-chloro-3-indolyl-β-D-glucopyrannoside ou le sel de monocyclohexylammonium du β-D-glucuronate de 5-bromo-4-chloro-3-indolyle.

37. Procédé selon l'une quelconque des revendications précédentes, où l'enzyme est la phosphatase alcaline, la β-D-galactosidase, une estérase, l'acétylchloline estérase, une alkylsulfatase, une sulfatase, la β-D-glucosidase ou la β-D-glucuronidase.

38. Procédé selon l'une quelconque des revendications précédentes, où le peroxyde d'hydrogène est dosé par colorimétrie, fluorimétrie ou de préférence chimioluminescence.

39. Procédé selon la revendication 38, où la technique est la colorimétrie et le réactif comprend du 1,2-diaminobenzène ou de l'acide 2,2'-aminobis(3-éthylbenzothiazoline-6-sulfonique).

40. Procédé selon la revendication 38, où la technique est la fluorimétrie, et le réactif comprend l'acide p-hydroxyphénylpropionique, la tyramine ou l'homovaline.

41. Procédé selon la revendication 38, où la technique est la chimioluminescence et le réactif est le luminol, l'isoluminol, le N-(4-aminobutyl)-N-éthylluminol ou un autre dérivé du luminol, la lucigénine, l'acridium, le pyrogallol, un oxalate, l'oxalate de bis(2,4,6-trichlorophényle), l'oxalate de bis(2,4-dinitrophényle), l'oxalate de pentafluorophényle ou un autre dérivé de type oxalate en association avec un colorant et un catalyseur d'oxydation, si cela est nécessaire.

42. Procédé selon la revendication 41, où le colorant comprend de l'acide 8-anilinonaphtalène-1-sulfonique, de la fluorescéine, de la rhodamine, du pyrène ou du chlorure de dansyle.

43. Procédé selon l'une quelconque des revendications précédentes, où l'enzyme est liée à un support.

44. Procédé selon la revendication 43, où le support est un anticorps, la substance à analyser ou une sonde oligonucléotidique.

45. Procédé selon l'une quelconque des revendications précédentes, appliqué à un liquide physiologique ou à une biopsie convenablement préparée.

46. Procédé selon l'une quelconque des revendications précédentes, où la substance à analyser est l'α-foetoprotéine, l'antigène carcinoembryonnaire, une hormone peptidique, une hormone stéroïde, une immunoglobuline ou un médicament.

47. Dispositif pour effectuer une analyse tel que défini dans la revendication 1 et comprenant un ou plusieurs esters 3-O-indoxyliques, une ou plusieurs enzymes appropriées à la conversion dudit ou desdits esters indoxyliques en l'indigo correspondant et un moyen pour déterminer le potentiel de production de peroxyde d'hydrogène.
